# EUROPÄISCHE PATENTSCHRIFT

(11) **EP 2 054 720 B1**
(45) Veröffentlichungstag und Bekanntmachung des Hinweises auf die Patenterteilung: **18.11.2009**
(21) Anmeldenummer: 07802742.2
(22) Anmeldetag: 21.08.2007
(51) Int. Cl.: G01N 33/487

(54) **DIAGNOSTISCHE BANDKASSETTE, INSBESONDERE FÜR BLUTZUCKERTESTS**
DIAGNOSTIC TAPE CASSETTE, PARTICULARLY FOR BLOOD SUGAR TESTS
CASSETTE DE RUBAN DE DIAGNOSTIC, EN PARTICULIER POUR DES TESTS DE GLYCEMIE

(30) Priorität: 22.08.2006 EP 06017404
(43) Veröffentlichungstag der Anmeldung: 06.05.2009
(73) Patentinhaber: F.HOFFMANN-LA ROCHE AG, 4070 Basel (CH); Roche Diagnostics GmbH, 68305 Mannheim (DE)
(72) Erfinder: SACHERER, Klaus-Dieter, 67281 Kirchheim (DE)
(74) Vertreter: Pfiz, Thomas
(86) Internationale Anmeldenummer: PCT/EP2007/058649
(87) Internationale Veröffentlichungsnummer: WO 2008/022999

(56) Entgegenhaltungen:
- DE-A1- 4 033 590
- DE-A1-102005 013 685
- US-A- 4 115 067
- US-A- 5 171 397
- US-A1- 2002 079 345
- US-A1- 2002 188 224

## Beschreibung

Die Erfindung betrifft eine diagnostische Bandkassette, insbesondere für Blutzuckertests, mit einem analytischen Testband, einer Vorratsspule zum Abspulen von unverbrauchtem Testband sowie einer Aufwickelspule zum Aufwickeln von verbrauchtem Testband, einem Gehäuse für die Spulen und einer Drehsicherung für die Aufwickelspule zumindest gegen ungewolltes Abwickeln von Testband.

Für die Selbstdiagnose von Diabetikern werden bislang in der Praxis einzelne Teststreifen eingesetzt, die nach der Applikation einer geringen Probenmenge photometrisch untersucht werden, um den Glucosegehalt in der Probe (Blut bzw. Gewebeflüssigkeit) möglichst genau und zuverlässig zu bestimmen. Zur Verbesserung der Anwenderfreundlichkeit wurde bereits vorgeschlagen, eine Vielzahl von Tests auf einem Testband in Form einer Bandkassette bereitzustellen. Solche Bandkassetten sollen sich als Einwegteil in kompakte Handgeräte einsetzen lassen, um alle erforderlichen Untersuchungsschritte automatisch und schnell ausführen zu können.

In diesem Zusammenhang ist in der DE 10 2005 013 685 eine Rücklaufsicherung gegen Bandabwickeln offenbart, um ein ungewolltes Ausspulen von gebrauchtem und mit Blut kontaminiertem Band zu verhindern. Die Sicherung soll dort in Form eines Freilaufgesperres ein Aufwickeln von Testband erlauben, während das Zurückdrehen in Gegenlaufrichtung gesperrt werden soll. Hierbei ist zu beachten, dass diagnostische Bandkassetten als Verbrauchsteile ein Massenprodukt darstellen, welches möglichst einfach herstellbar sein soll und zuverlässig und mit hoher Benutzerfreundlichkeit arbeiten soll.

Ausgehend hiervon liegt der Erfindung die Aufgabe zugrunde, die im Stand der Technik vorgeschlagenen Testbandsysteme weiter zu verbessern und bei einfacher Herstellbarkeit besondere Anwendungsvorteile zu erreichen.

Zur Lösung dieser Aufgabe wird die im unabhängigen Patentanspruch angegebene Merkmalskombination vorgeschlagen. Vorteilhafte Ausgestaltungen und Weiterbildungen der Erfindung ergeben sich aus den abhängigen Ansprüchen.

Das Erfindungskonzept liegt darin, dass die Drehsicherung eine an dem Gehäuse angebrachte, axial gegen die Aufwickelspule vorgespannte Sperrfeder aufweist, welche über mindestens eine Federklinke mit einer stirnseitig an der Aufwickelspule angeordneten Freilaufverzahnung in permanentem Zahneingriff gegen Ausspulen einer Bandschlaufe steht. Damit wird nicht nur der Schutz gegen Manipulation erhöht, sondern es kann auch stets ein ungewolltes Ausspulen einer Bandschlaufe etwa durch Erschütterungen verhindert und damit eine definierte Bandpositionierung an einer Sensorstelle eingehalten werden.

Vorteilhafterweise ist die Sperrfeder vorzugsweise als konzentrisches Ringgebilde oder Spiralfeder an einer Wand des Gehäuses angeformt, wobei eine von der Wand abstehende Innenpartie der Sperrstellfeder die mindestens eine Sperrklinke trägt.

Um den möglichen Rücklaufwinkel bei gegebener Zähnezahl zu minimieren, ist es vorteilhaft, wenn die Federklinke als Mehrfachklinke zur Unterteilung der Sperrstellungen der Freilaufverzahnung ausgebildet ist, so dass die Einzelklinken über die Zahnteilung der Freilaufverzahnung versetzt in Sperreingriff kommen.

Eine besonders günstige Anordnung sieht vor, dass die Freilaufverzahnung an einer der Sperrfeder zugewandten Stirnwand eines Spulenkörpers der Aufwickelspule angeformt ist, und dass der Spulenkörper über einen ringförmigen Bund an dem Gehäuse vorzugsweise über eine Sperrverzahnung abgestützt ist.

Um eine drehrichtungsabhängige Sperrwirkung zu erreichen, ist es günstig, wenn die Freilaufverzahnung durch einen Zahnkranz mit unsymmetrischem Zahnprofil gebildet ist, wobei die flacheren Zahnflanken bei Drehung in Aufwickelrichtung eine Gleitschräge für die Federklinke bilden und die steileren Zahnflanken bei Drehung in Abwickelrichtung die Federklinke formschlüssig abstützen.

Für eine Reibungsminimierung ist es vorteilhaft, wenn die Zähne der Freilaufverzahnung eine über ihre Breite abfallende Zahnhöhe aufweisen, so dass die Eingriffslänge der Federklinke verkürzt wird.

Gegenstand der Erfindung ist auch ein Testsystem umfassend ein Testgerät und eine darin eingesetzte diagnostische Bandkassette insbesondere für Blutzuckertests, wobei die Bandkassette ein analytisches Testband, eine Vorratsspule zum Abspulen von unverbrauchtem Testband sowie eine Aufwickelspule zum Aufwickeln von verbrauchtem Testband, ein Gehäuse für die Spulen und eine erfindungsgemäße Drehsicherung für die Aufwickelspule zumindest gegen ungewolltes Abwickeln von Testband aufweist.

Bei einem solchen System ist es von besonderem Vorteil, wenn beim Einsetzen der Bandkassette eine Antriebsspindel des Testgeräts in die Aufwickelspule eingreift und dabei das Ausheben der Sperrverzahnung oder eine Aufhebung des Reibschlusses des Reibelements in die Freigabestellung bewirkt. Eine weitere Verbesserung kann dadurch erreicht werden, dass die Antriebsspindel über eine Feder in Richtung einer Drehachse verstellbar ist und unter Federhub in die Aufwickelspule eingreift. Hierbei ist es von Vorteil, wenn die Antriebsspindel in einer Mitnahmebohrung der Aufwickelspule über mindestens einen Mitnehmer eine Drehbewegung überträgt.

Es zeigen:
- Fig. 1: eine Bandkassette für Blutzuckertests mit einer Abwickelsicherung für das Testband in einer perspektivischen Darstellung;
- Fig. 2: eine Aufwickelspule der Bandkassette mit einer Sperrverzahnung in vergrößerter perspektivischer Ansicht;
- Fig. 3: einen Ringflansch der Bandkassette mit einer Gegenverzahnung in der Draufsicht;
- Fig. 4 und 5: die Aufwickelspule und den Ringflansch in einer Eingriffstellung und einer Freigabestellung der Sperrverzahnung im Axialschnitt;
- Fig. 6: ein tragbares Messgerät mit einer Aufnahme für die Bandkassette in einer perspektivischen Darstellung;
- Fig. 7: eine Antriebsspindel des Geräts nach Fig. 6 in der Seitenansicht;
- Fig. 8: den Gehäusedeckel mit angeformter Sperrfeder in einer erfindungsgemäßenAusführungsform der Bandkassette;
- Fig. 9: eine mit einer erfindungsgemäßen stirnseitigen Freilaufverzahnung versehene Aufwickelspule in der Seitenansicht;
- Fig. 10: die Sperrfeder nach Fig. 8 in Eingriff mit der Freilaufverzahnung nach Fig. 9 für eine permanente Schlaufensperre in einer abgebrochenen perspektivischen Ansicht;
- Fig. 11 bis 13: eine Alternative zur Sperrverzahnung, mit einer Aufwickelspule gemäß Fig. 11 für einen Reibeingriff an einem gehäuseseitigen Reibring gemäß Fig. 12 oder 13 in perspektivischer Darstellung.

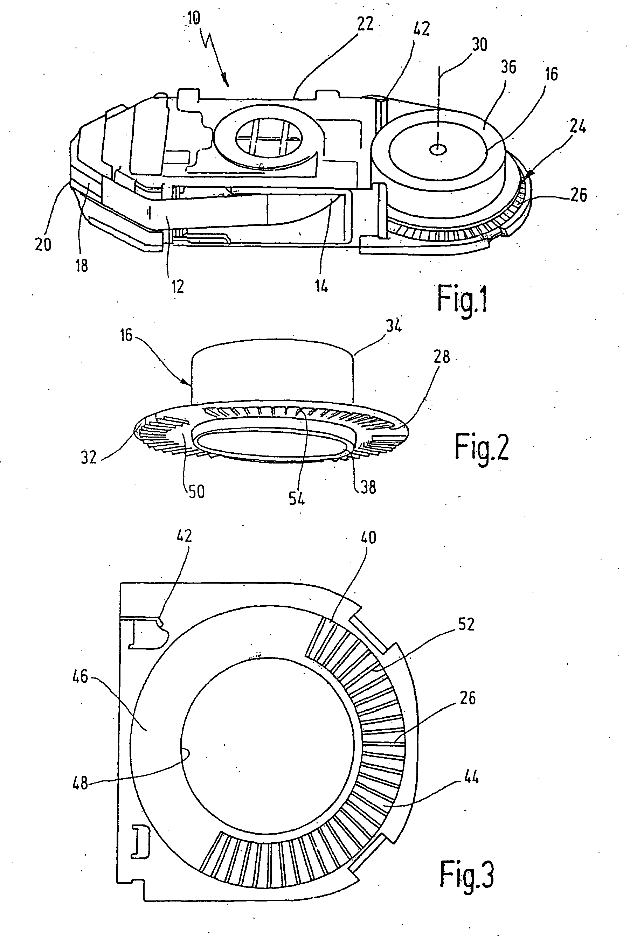

Die in Fig. 1 bei abgenommenem Deckel dargestellte Bandkassette ermöglicht die Durchführung einer Vielzahl von Glucoseuntersuchungen an vor Ort vom Patienten selbst gewonnenen Blutproben. Zu diesem Zweck umfasst die Bandkassette 10 ein analytisches Testband 12, das von einer Vorratsspule 14 abziehbar und auf eine Aufwickelspule 16 aufwickelbar ist, wobei ein Testfeld 18 an einer Applikationsspitze 20 umgelenkt wird, um eine vorderseitige Applikation von Körperflüssigkeit (Blut bzw. Gewebeflüssigkeit) und eine rückseitige reflektometrische Vermessung zu erlauben. Die abschnittsweise auf dem Testband 12 aufgebrachten Testfelder 18 enthalten Trockenchemikalien, die auf den Analyten (Glucose) in der applizierten Blutflüssigkeit ansprechen und bei rückseitiger Beleuchtung zu einer messbaren Veränderung der Lichtrückstrahlung führen.

Die das Testband 12 tragenden Spulen 14, 16 sind in ein Gehäuse 22 der Kassette 10 eingesetzt, in welchem bei aufgesetztem Deckel nur die Applikationsspitze 20 offen zugänglich ist. Um bei Manipulationen von außen ein ungewolltes Abwickeln von verbrauchtem und mit Blut kontaminiertem Testband zu verhindern, ist zwischen Aufwickelspule 16 und Gehäuse 22 ein Drehgesperre 24 vorgesehen. Dieses ist durch jeweils eine an dem Gehäuse 22 und der Aufwickelspule 16 ausgebildete Sperrverzahnung 26, 28 gebildet, welche durch eine Axialbewegung der Aufwickelspule längs der Spulenachse 30 zwischen einer gegenseitigen Eingriffbestellung und einer Freigabestellung schaltbar ist.

Wie am besten aus Fig. 2 ersichtlich, weist die Aufwickelspule 16 eine stirnseitig verzahnte Zahnscheibe 32 auf, welche die Sperrverzahnung 28 nach Art eines Kronenrades trägt. Die Zahnscheibe 32 ist durch einen Bund eines mit dem Testband 12 umwickelbaren hohlzylindrischen Spulenkörpers 34 gebildet, wobei die Sperrverzahnung 28 von dem Testbandwickel 36 (Fig. 1) abgewandt ist. An der verzahnten Seite besitzt die Aufwickelspule 16 einen Ringfortsatz 38 für eine schwimmende Zentrierung in der Gegenverzahnung 26.

Wie in Fig. 3 gezeigt, besitzt das Gehäuse 22 einen Ringflansch 40, welcher die gehäuseseitige Sperrverzahnung 26 trägt. Diese ist an einem von der in Bandtransportrichtung gesehen letzten Umlenkstelle 42 für das aufzuwickelnde Testband 12 abgewandten Ringsegment 44 ausgebildet, während das verbleibende Ringsegment 46 unverzahnt ist, so dass bei einer Verkippung aufgrund starker Bandzugbelastung der Aufwickelspule 16 ein ungewolltes Sperren vermieden wird. Der Ringflansch 40 begrenzt eine Gehäuseöffnung 48, in welcher der Ringfortsatz 38 der Aufwickelspule 16 in der Eingriffstellung mit Querspiel 49 gelagert ist. Dieses Querspiel 49 wird dadurch erreicht, dass die Gehäuseöffnung 48 einen um ca. 0,5 mm größeren Durchmesser als der Ringfortsatz 38 an seinem Umfang besitzt. Die Sperrverzahnungen 26, 28 erlauben aufgrund ihrer radialen Erstreckung eine entsprechende Querauslenkung der Aufwickelspule 16, wodurch Positioniertoleranzen ausgeglichen werden können.

Die Sperrverzahnungen 26, 28 sind somit an einander zugewandten Flächen 44, 50 ringförmig ausgebildet, so dass die Aufwickelspule 16 in einer beliebigen Drehstellung auf den Ringflansch 40 aufsetzbar ist. Um die Sperrwirkung gegen Rückdrehen zu verbessern, weisen die Zähne der Sperrverzahnungen 26, 28 unsymmetrische Zahnflanken auf, wobei die beim Rückdrehen der Aufwickelspule 16 in der Eingriffstellung gegeneinander anschlagenden Zahnflanken 52, 54 steiler als die jeweils gegenüber liegenden Zahnflanken sind.

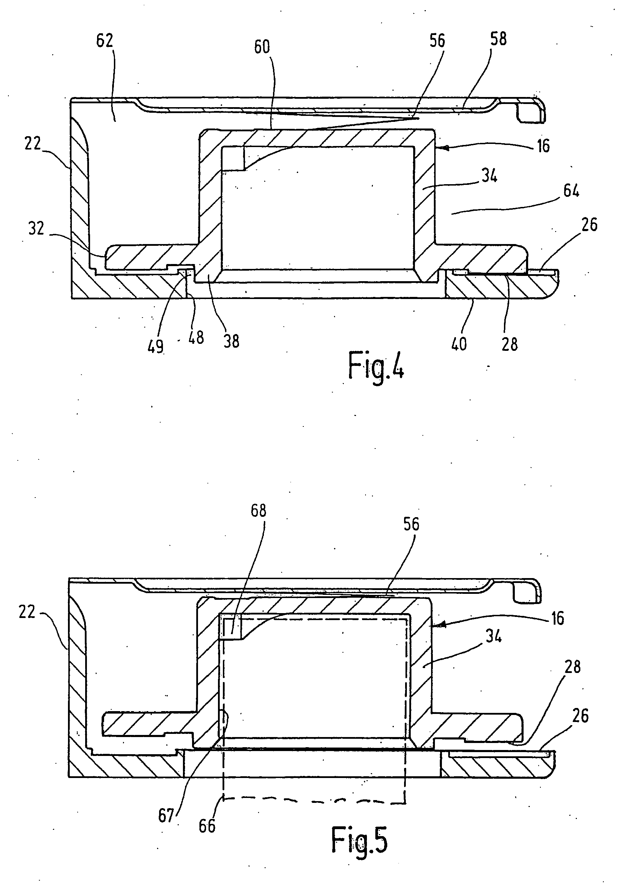

Die Schaltfunktion des Drehgesperres 24 ist am besten aus Fig. 4 und 5 ersichtlich. Bei Aufbewahrung oder geräteunabhängiger Handhabung der Bandkassette 10 stehen die Sperrverzahnungen 26, 28 in gegenseitigem Eingriff, so dass durch Formschluss beide Drehrichtungen gesperrt sind (Fig. 4). Hierfür sorgt eine Rückstellfeder 56, welche zwischen dem Deckel 58 des Gehäuses 22 und der geschlossenen Stirnfläche 60 des Spulenkörpers 34 eingespannt ist und diesen gegen den Ringflansch 40 drängt. Zweckmäßig ist die Rückstellfeder 56 als Spiralfeder an dem Deckel 58 angeformt. Die Aufwickelspule 16 lässt sich dann in die Gehäusekammer 62 einsetzen, wobei keine materielle Drehachse erforderlich ist und der Ringraum 64 zwischen dem Deckel 58 und dem Drehgesperre 24 zur Aufnahme des Testbands 12 freigehalten ist.

Bei Gebrauch der Bandkassette 10 greift eine in Fig. 5 nur schematisch gezeigte Antriebsspindel 66 eines geräteseitigen Antriebs in die zentrale Mitnahmebohrung 67 des Spulenkörper 34 axial ein und bewirkt dadurch unter Kompression der Feder 56 ein Ausheben in die Freigabestellung. Hierbei treten die Sperrverzahnungen 26, 28 außer Eingriff, und die Aufwickelspule 16 kann somit frei gedreht werden. Die Antriebsspindel 66 übernimmt dann ggf. die Sperrfunktion in Abwickelrichtung, während das Aufwickeln von Testband - in dem gezeigten Beispiel bei Drehung im Uhrzeigersinn - über drei in Drehrichtung verteilte Mitnehmer 68 ermöglicht wird. Aufgrund der im Abstand befindlichen Sperrverzahnungen 26, 28 werden so störende Geräusche vermieden, und es treten keine unerwünschten Bremsmomente auf.

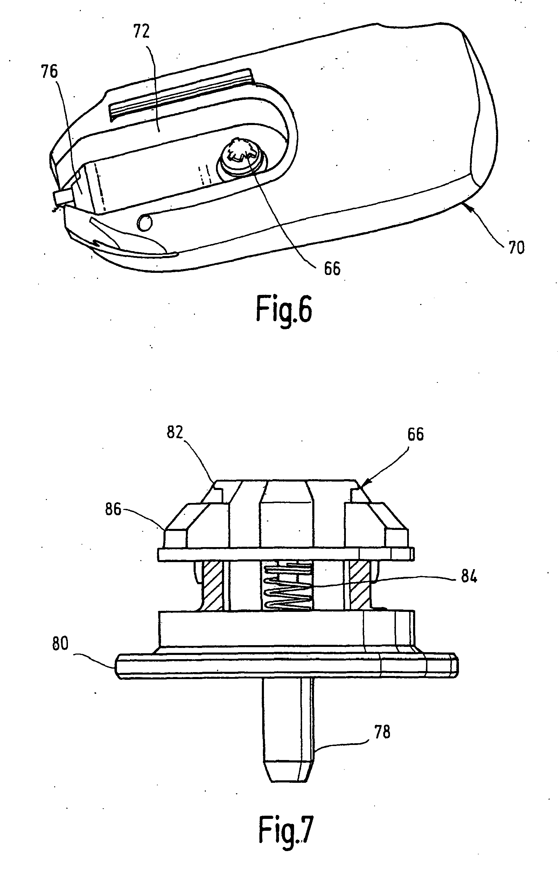

Fig. 6 zeigt ein Handgerät 70 mit einer Aufnahme 72, in die sich die Bandkassette 10 einsetzen lässt, um mit dem so gebildeten Messsystem einen weitgehend automatischen Messablauf zu ermöglichen. In der Aufnahme 72 wird die geräteseitige Messoptik 76 relativ zu der Applikationsspitze 20 positioniert. Hierbei erlaubt das vorstehend erläuterte Querspiel der Aufwickelspule 16 einen Ausgleich von Montage- und Positioniertoleranzen der eigens in dem Gerätegehäuse montierten Antriebsspindel 66.

Die in Fig. 7 gesondert gezeigte Antriebsspindel 66 besitzt einen die Aufnahme 72 bodenseitig durchsetzenden Abtriebszapfen 78, der über ein Zahnradgetriebe mit einem Antriebsmotor in dem Gerät 70 gekoppelt ist (nicht gezeigt). Auf dem Zapfen 78 sitzt eine Stützscheibe 80, auf welcher wiederum ein Kupplungskopf 82 über eine Schraubendruckfeder 84 bezüglich des Zapfens 78 axialbeweglich abgestützt ist. Beim Einsetzen der Kassette 10 in die Aufnahme 72 taucht der Kupplungskopf 82 in die Mitnahmebohrung 67 der Aufwickelspule 16 ein und kommt mit seinen Umfangsnocken 86 unter der Kraft der Feder 84 in Formschluss mit den Mitnehmern 68, so dass ein Drehmoment übertragen werden kann.

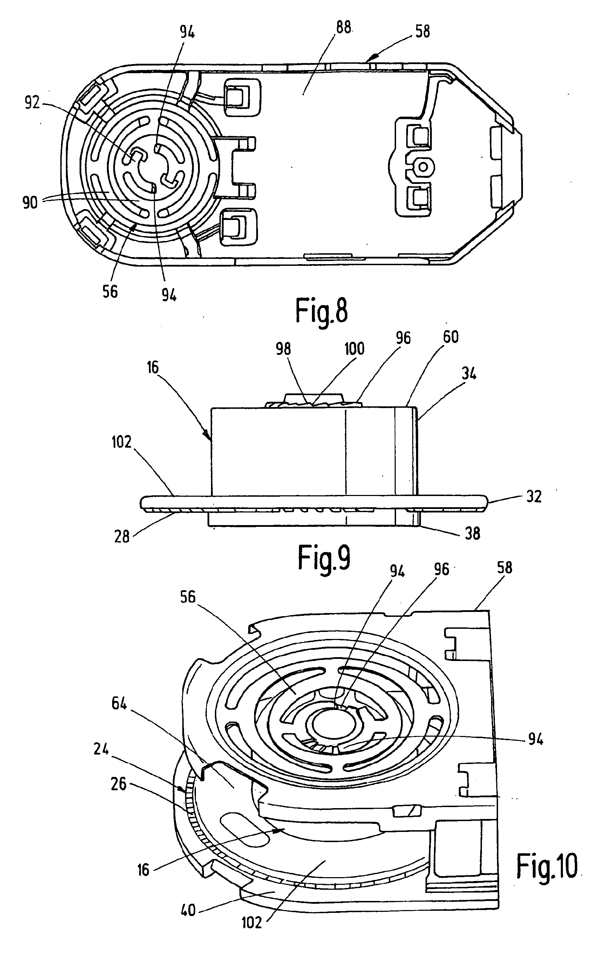

Bei den im Folgenden beschriebenen Ausführungsformen des Erfindung wurden für gleiche Teile gleiche Bezugszeichen wie oben erläutert verwendet. Das Beispiel nach Fig. 8 bis 10 unterscheidet sich im Wesentlichen dadurch, dass als permanente Sicherung gegen ungewolltes Bandausspulen eine Sperrfeder 56 am Gehäusedeckel 58 in Kombination mit einer Freilaufverzahnung an der Aufwickelspule 16 vorgesehen ist. Dadurch wird in jedem Gebrauchszustand das Abwickeln einer Bandschlaufe vermieden.

Wie in Fig. 8 gezeigt, ist die Sperrfeder 56 als Ringgebilde aus dem Gehäuseblech 88 ausgestanzt, wobei zwei koaxiale Ringe 90 durch Brücken miteinander verbunden sind. An dem inneren Ring sind zwei Ringsegmente 92 angeformt, die mit jeweils einem zum Gehäuseinneren abgebogenen Ende eine Sperrklinke 94 bilden, während das andere Segmentstück in einer die Feder 56 rückseitig überdeckenden Kunststoffumspritzung gehalten ist. Die Ringsegmente 92 und Ringe 90 sind axial so gegeneinander versetz, dass sich eine gegen die Stirnfläche 60 der eingesetzten Aufwickelspule 16 ausgewölbte Drückfederanordnung ergibt (vgl. Fig. 10).

Fig. 9 zeigt eine entsprechend angepasste Aufwickelspule 16, die im Unterschied zu der Ausführung nach Fig. 2 stirnseitig mit der Freilaufverzahnung 96 versehen ist. Diese wird durch einen Zahnkranz mit unsymmetrischem Zahnprofil gebildet, wobei die flacheren Zahnflanken 98 bei Drehung in Aufwickelrichtung eine Gleitschräge für die Federklinke 94 bilden und die steileren Zahnflanken 100 bei Drehung in Abwickelrichtung die Federklinke 94 formschlüssig sperren.

Fig. 10 veranschaulicht die drehrichtungsabhängige Wirkung des Federeingriffs. Bei eingesetzter Aufwickelspule 16 stehen die nach unten abgebogenen Sperrklinken 94 unter Federvorspannung in Zahneingriff, so dass in der gezeigten Stellung eine Klinke gegen eine steile Zahnflanke 100 anschlägt, während die andere Klinke mittig auf einer flachen Zahnflanke 98 aufliegt. Auf diese Weise ergibt sich eine Halbierung der wirksamen Zahnteilung, so dass bei weiter und stabiler Verzahnung dennoch ein geringer Rücklaufwinkel bis zum Eintritt der Sperrwirkung erreicht wird. Zur Reibungsverringerung können die Zähne der ringförmigen Freilaufverzahnung 96 eine nach innen über ihre Breite abfallende Zahnhöhe aufweisen, wodurch die Eingriffslänge der Federklinken 94 verkürzt wird.

Um eine zusätzlich Sperrwirkung bei Nichtgebrauch in beide Drehrichtungen zu erhalten, kann der Bund 102 des Spulenkörpers 34 an seiner Auflageseite mit einer umlaufenden Sperrverzahnung 28 versehen sein, die in eine Gegenverzahnung 26 am Gehäuseflansch 40 eingreift. Dieses Drehgesperre 24 wird beim Einsetzen in das Gerät 70 durch die Antriebsspindel 66 ausgehoben, während die richtungsabhängige Sperrwirkung der Drehsicherung 94, 96 unter höherer Federvorspannung, aber geringerem Eingriffswinkel der Federklinken erhalten bleibt.

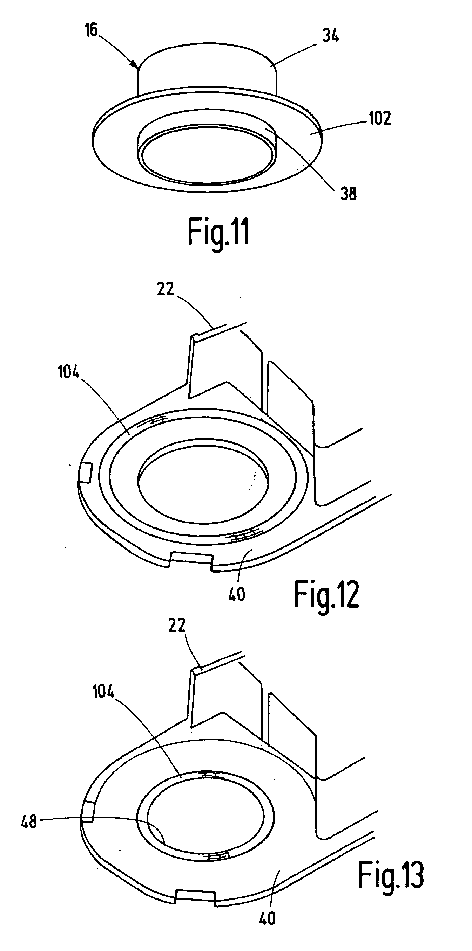

In Fig. 11 bis 13 ist anstelle der Sperrverzahnung 26, 28 eine von der Drehstellung und Drehrichtung unabhängige Reibschlusssperre vorgesehen. Gemäß Fig. 11 bleibt hierbei der Bund 102 der Aufwickelspule 16 frei von einer Verzahnung. Für den Reibschluss ist an der Auflagefläche des Ringflansches 40 des Gehäuses 22 ein Reibring 104 angeformt, der entweder etwa in der Ringflächenmitte (Fig. 12) oder innen an dem die Flanschöffnung 48 begrenzenden Rand (Fig. 13) sitzt. Der Reibring 104 besteht aus einem thermoplastischen Elastomermaterial (TPE), das im selben Fertigungsschritt zusammen mit Dichtungskomponenten an das Gehäuse 22 im Spritzgießverfahren angespritzt wird. Auch hier wird der Reibschluss durch eine am Gehäuse 22 ausgebildete Rückstellfeder 56 entsprechend Fig. 4 unterstützt, während beim Eingriff der Antriebsspindel 66 entsprechend Fig. 5 die Aufwickelspule 16 entgegen der Federkraft angehoben und dabei der Reibschluss aufgehoben wird.

## Patentansprüche

1. Diagnostische Bandkassette, insbesondere für Blutzuckertests, mit einem analytischen Testband (12), einer Vorratsspule (14) zum Abspulen von unverbrauchtem Testband (12) sowie einer Aufwickelspule (16) zum Aufwickeln von verbrauchtem Testband (12), einem Gehäuse (22) für die Spulen (14,16) und einer Drehsicherung (24) für die Aufwickelspule (16), **dadurch gekennzeichnet, dass** die Drehsicherung (24) eine an dem Gehäuse (22) angebrachte, axial gegen die Aufwickelspule (16) vorgespannte Sperrfeder (56) aufweist, welche über mindestens eine Federklinke (94) mit einer stirnseitig an der Aufwickelspule (16) angeordneten Freilaufverzahnung (96) in permanentem Zahneingriff gegen Ausspulen einer Bandschlaufe steht.

2. Bandkassette nach Anspruch 1, **dadurch gekennzeichnet, dass** die Sperrfeder (56) vorzugsweise als konzentrisches Ringgebilde oder Spiralfeder an einer Wand (58) des Gehäuses (22) angeformt ist, wobei eine von der Wand (58) abstehende Innenpartie der Sperrfeder die mindestens eine Federklinke (94) trägt.

3. Bandkassette nach Anspruch 1 oder 2, **dadurch gekennzeichnet, dass** die Federklinke (94) als Mehrfachklinke zur Unterteilung der Sperrstellungen der Freilaufverzahnung (96) ausgebildet ist, so dass die Einzelklinken über die Zahnteilung der Freilaufverzahnung (96) versetzt in Sperreingriff kommen.

4. Bandkassette nach einem der Ansprüche 1 bis 3, **dadurch gekennzeichnet, dass** die Freilaufverzahnung (96) an einer der Sperrfeder (56) zugewandten Stirnwand eines Spulenkörpers (34) der Aufwickelspule (16) angeformt ist, und dass der Spulenkörper (34) über einen ringförmigen Bund (102) an dem Gehäuse (22) vorzugsweise über eine Sperrverzahnung (26,28) abgestützt ist.

5. Bandkassette nach einem der Ansprüche 1 bis 4, **dadurch gekennzeichnet, dass** die Freilaufverzahnung (96) durch einen Zahnkranz mit unsymmetrischem Zahnprofil gebildet ist, wobei die flacheren Zahnflanken(98) bei Drehung in Aufwickelrichtung eine Gleitschräge für die Federklinke (94) bilden und die steileren Zahnflanken zwei Drehung in Abwickelrichtung die Federklinke (94) formschlüssig sperren.

6. Bandkassette nach einem der Ansprüche 1 bis 5, **dadurch gekennzeichnet, dass** die Zähne der Freilaufverzahnung (96) eine über ihre Breite abfallende Zahnhöhe aufweisen, so dass die Eingriffslänge der Federklinke (94) verkürzt wird.

7. Testsystem umfassend ein Testgerät und eine darin eingesetzte diagnostische Bandkassette (10) insbesondere für Blutzuckertests, wobei die Bandkassette (10) ein analytisches Testband (12), eine Vorratsspule (14) zum Abspulen von unverbrauchtem Testband (12) sowie eine Aufwickelspule (16) zum Aufwickeln von verbrauchtem Testband (12), ein Gehäuse (22) für die Spulen (14,16) und eine Drehsicherung (24) für die Aufwickelspule (16) zumindest gegen ungewolltes Abwickeln von Testband (12) nach Anspruch 1 aufweist.

8. Testsystem nach Anspruch 7, **dadurch gekennzeichnet, dass** beim Einsetzen der Bandkassette (10) eine Antriebsspindel (66) des Testgeräts in die Aufwickelspule (16) eingreift

9. Testsystem nach Anspruch 8, **dadurch gekennzeichnet, dass** die Antriebsspindel unter Axialbewegung der Aufwickelspule gegen eine Rückstellfeder ein Ausheben der Sperrverzahnung (26,28) oder eine Aufhebung des Reibschlusses des Reibelements (104) in die Freigabestellung bewirkt.

10. Testsystem nach Anspruch 8 oder 9, **dadurch gekennzeichnet, dass** die Antriebsspindel (66) über eine Feder (84) in Richtung einer Drehachse verstellbar ist und unter Federhub in die Aufwickelspule (16) eingreift.

11. Testsystem nach einem der Ansprüche 8 bis 10, **dadurch gekennzeichnet, dass** die Antriebsspindel (66) in einer Mitnahmebohrung (67) der Aufwickelspule (16) über mindestens einen Mitnehmer (68) eine Drehbewegung überträgt.

## Claims

1. Diagnostic tape cassette, particularly for blood sugar tests, comprising an analytical test tape (12), a supply spool (14) for winding off unused test tape (12) and a take-up spool (16) for winding used test tape (12), a housing (22) for the spools (14, 16) and a rotational lock (24) for the take-up spool (16), **characterized in that** the rotational lock (24) has a catch spring (56) attached to the housing (22) and axially pretensioned against the take-up spool (16) which, by means of at least one spring latch (94), permanently meshes with free-wheel teeth (96) arranged on the front of the take-up spool (16) to prevent against unwinding of a tape loop.

2. Tape cassette according to claim 1, **characterized in that** the catch spring (56) is preferably formed on a wall (58) of the housing (22) as a concentric ring structure or spiral spring where an inner member of the catch spring projecting from the wall (58) carries the at least one spring latch (94).

3. Tape cassette according to claim 1 or 2, **characterized in that** the spring latch (94) is designed as a multiple latch to subdivide the locked positions of the free-wheel teeth (96) such that the individual latches are brought into a locking engagement which is displaced over the tooth pitch of the free-wheel teeth (96).

4. Tape cassette according to one of the claims 1 to 3, **characterized in that** the free-wheel teeth (96) are formed on an end wall of a spool body (34) of the take-up spool (16) which faces the catch spring (56) and that the spool body (34) is supported on the housing (22) by means of an annular collar (102) and preferably by locking teeth (26, 28).

5. Tape cassette according to one of the claims 1 to 4, **characterized in that** the free-wheel teeth (96) are formed by a crown gear with an asymmetric tooth profile where the flatter tooth flanks (98) form a slide slope for the spring latch (94) during rotation in the winding direction and the steeper tooth flanks (100) block the spring latch (94) in a form-fitting manner during rotation in the unwinding direction.

6. Tape cassette according to one of the claims 1 to 5, **characterized in that** the teeth of the free-wheel teeth (96) have a tooth height which decreases over their width thus shortening the engagement length of the spring latch (94).

7. Test system comprising a test device and a diagnostic tape cassette (10) inserted therein in particular for blood sugar tests where the tape cassette (10) has an analytical test tape (12), a supply spool (14) for winding off unused test tape (12) and a take-up spool (16) for winding used test tape (12), a housing (22) for the spools (14, 16) and a rotational lock (24) for the take-up spool (16) at least against inadvertent unwinding of test tape (12) according to claim 1.

8. Test system according to claim 7, **characterized in that** a drive spindle (66) of the test device engages in the take-up spool (16) when the tape cassette (10) is inserted.

9. Test system according to claim 8, **characterized in that** when the take-up spool is moved axially against a return spring, the drive spindle lifts out the locking teeth (26, 28) or releases the frictional connection of the friction element (104) into the release position.

10. Test system according to claim 8 or 9, **characterized in that** the drive spindle (66) can be adjusted by a spring (84) in the direction of an axis of rotation and engages in the take-up spool (16) under spring displacement.

11. Test system according to one of the claims 8 to 10, **characterized in that** the drive spindle (66) in a driver bore (67) of the take-up spool (16) transfers a rotary movement by means of at least one driver (68).

## Revendications

1. Cassette de ruban de diagnostic, en particulier pour des tests de glycémie, ayant un ruban de test analytique (12), une bobine débitrice (14) pour débiter du ruban de test inutilisé (12) ainsi qu'une bobine réceptrice de ruban de test utilisé (12), un logement (22) pour les bobines (14, 16) et un dispositif de blocage en rotation (24) pour la bobine réceptrice (16), **caractérisée en ce que** le dispositif de blocage en rotation (24) présente un ressort d'arrêt (56) appliqué au logement (22), précontraint axialement contre la bobine réceptrice (16), lequel ressort se trouve en engrènement permanent par l'intermédiaire d'au moins un cliquet à ressort (94) avec une denture mobile libre (96) disposé frontalement sur la bobine réceptrice (16) contre le dévidage d'une boucle de ruban.

2. Cassette de ruban selon la revendication 1, **caractérisée en ce que** le ressort d'arrêt (56) est de préférence formé en tant que structure annulaire concentrique ou en tant que ressort en spirale sur une paroi (58) du logement (22), une partie intérieure du ressort d'arrêt, éloignée de la paroi (58) portant ledit au moins un cliquet à ressort (94).

3. Cassette de ruban selon la revendication 1 ou 2, **caractérisée en ce que** le cliquet à ressort (94) est formé en tant que cliquet multiple pour subdiviser les positions d'arrêt de la denture mobile libre (96), de sorte que les cliquets individuels viennent de façon décalée en engrènement d'arrêt par l'intermédiaire du pas des dents de la denture mobile libre (96).

4. Cassette de ruban selon l'une quelconque des revendications 1 à 3, **caractérisée en ce que** la denture mobile libre (96) est formée sur une paroi frontale tournée vers le ressort d'arrêt (56), d'un corps de bobine (34) de la bobine réceptrice (16), et que le corps de bobine (34) est supporté par une attache de forme annulaire (102) sur le logement (22), de préférence par une denture d'arrêt (26, 28).

5. Cassette de ruban selon l'une quelconque des revendications 1 à 4, **caractérisée en ce que** la denture mobile libre (96) est formée par une couronne de dents avec un profil de dents non symétrique, les flancs de dent plats (98) forment une inclinaison de glissement pour le cliquet à ressort (94) lors d'une rotation dans la direction d'enroulement et les flancs de dent (100) à forte pente bloquent mécaniquement le cliquet à ressort (94) lors d'une rotation dans la direction de dévidage.

6. Cassette de ruban selon l'une quelconque des revendications 1 à 5, **caractérisée en ce que** les dents de la denture mobile libre (96) présentent une hauteur de dent diminuant sur sa largeur, de sorte que la longueur d'engrènement du cliquet à ressort (94) est raccourcie.

7. Système de test comprenant un appareil de test et une cassette de ruban (10) de diagnostic mise en place dans celui-ci, en particulier pour des tests de glycémie, la cassette de ruban (10) présentant un ruban de test analytique (12), une bobine débitrice (14) pour dévider du ruban de test inutilisé (12) ainsi qu'une bobine réceptrice (16) pour enrouler du ruban de test utilisé (12), un logement (22) pour les bobines (14, 16) et un dispositif de blocage en rotation (24) pour la bobine réceptrice (16) au moins contre un dévidage non voulu du ruban de test (12) selon la revendication 1.

8. Système de test selon la revendication 7, **caractérisé en ce que**, lors de la mise en place de la cassette de ruban (10), une broche d'entraînement (66) de l'appareil de test s'engrène dans la bobine réceptrice (16).

9. Système de test selon la revendication 8, **caractérisé en ce que** la broche d'entraînement avec le mouvement axial de la bobine réceptrice contre un ressort de rappel, provoque un soulèvement de la denture d'arrêt (26, 28) ou une suppression du frottement d'un élément frottant (104) dans la position de déblocage.

10. Système de test selon la revendication 8 ou 9, **caractérisé en ce que** la broche d'entraînement (66) est ajustable par l'intermédiaire d'un ressort (84) dans la direction d'un axe de rotation et s'engrène dans la bobine réceptrice (16) avec soulèvement du ressort.

11. Système de test selon l'une quelconque des revendications 8 à 10, **caractérisé en ce que** la broche d'entraînement (66), dans un alésage d'entraînement (67) de la bobine réceptrice (16), communique un mouvement de rotation par l'intermédiaire d'au moins un tenon d'entraînement (68).
